# EUROPEAN PATENT APPLICATION

(11) **EP 1 416 049 A1**
(43) Date of publication of application: **06.05.2004**
(21) Application number: 02743278.0
(22) Date of filing: 28.06.2002
(51) Int. Cl.: C12N 15/29, C12N 15/63, C12N 15/87

(54) **METHOD OF INTERFERING WITH A VIRUS INFECTION IN PLANTS**

(30) Priority: 06.07.2001 ES 200101593
(71) Applicant: CONSEJO SUPERIOR DE INVESTIGACIONES CIENTIFICAS, 28006 Madrid (ES)
(72) Inventor: TENLLADO PERALO, Francisco, Ctr. Inv. Biologicas, E-28006 Madrid (ES); DIAZ RUIZ, Jose, Ramon, Ctr. Inv. Biologicas, E-28006 Madrid (ES)
(74) Representative: Ungria Lopez, Javier
(86) International application number: PCT/ES2002/000319
(87) International publication number: WO 2003/004649

(57) **Abstract**

The invention relates to a method for interfering with or degrading in a specific manner (preferably by means of genic silencing) any nucleotide sequence of single-strand RNA (ssRNA) (preferably messenger RNA of viruses, of endogenous genes or of transgenes) in the cells of any plant, which method consists of the direction application in any plant tissue (preferably leaves) and by any application method (preferably inoculation or infiltration) of double-strand RNA molecules (dsRNA) produced by any natural or synthetic procedure (preferably by means of in vitro transcription) whose dsRNA molecules have sufficient sequence similarity with any fragment of ssRNA that it is wished to degrade, or whose expression it is wished to silence, in the treated plant.

## Description

### Field of the invention

The present invention refers to the use of double-strand RNA molecules (hereinafter referred to as dsRNA) for interfering in a specific manner with viral infections in plants. More particularly, the invention refers to the preparation of molecules of dsRNA of a defined sequence and their direct application in any plant in order to suppress the multiplication in the treated plant of any virus having sufficient sequence similarity with dsRNA.

### State of the art

Plant viruses represent one of the most important groups of pathogens, since they cause serious economic losses in many species of agricultural interest throughout the world. The majority of known viruses infecting plants have genomes consisting of single-strand RNA (hereinafter referred to as ssRNA) and messenger sense RNA (hereinafter referred to mRNA) as genetic material. These genomes are relatively simple, coding solely between 4 and 10 proteins, depending on the virus group. These proteins are involved in basic aspects of the biological cycle of these pathogens, such as the replication process of viral RNA, the movement of the virus from cell to cell or the translocation of the viral progeny via the host plant (Matthews, Plant Virology, Third Edition (San Diego, *Academic Press, Inc.)* (1991).

Owing to the limited quantity of genetic information that these pathogens contain, and to the complexity of the infective process, the viruses depend on the cell machinery of the host for many stages of their infection cycle. Many of those factors of the host are usual components of the transcriptional or translational apparatus of the cell, exploited by the virus for carrying out an integral or regulating function in the replication and diffusion of viral RNA in the plant (Lai, Virology, 244, 1 (1998). It can be said that the host and parasite are intimately inter-linked and compete in the molecular mechanisms governing their biological cycles. The success or failure of viral infections is the final consequence of how the viruses and their hosts interact, in other words, of the defensive strategies of the plant and the counterattack response on the part of the viruses (Carrington and Whitham, *Current Opinion in Plant Biology,* 1, 336 (1998).

Control over viral diseases in crops presents great difficulties given that, for the majority of viruses, there are no commercial varieties of crop available that can carry resistance genes against the pathogen, incorporated by means of traditional genetic improvement procedures. Moreover, in some cases wherein they have been obtained, the resistances are overcome by the appearance of new virus strains (Matthews, Plant Virology, Third Edition (San Diego, *Academic Press, Inc.)* (1991). On the other hand, the use of insecticides and/or chemical agents which have been employed for controlling the different vectors through which viral infections are spread in nature raises serious problems of environmental contamination. Therefore, owing to the losses that plant viruses occasion in economically important crops and the difficulties presented by their control, efforts currently being made are focused on studying the virus-plant interaction, especially in processes taking place during the biological cycle of these pathogens, as well as the defence mechanisms unleashed by the plants against viral infections. The final objective of these studies is in all cases the control of viral diseases in plants by means of developing new strategies for interfering with or blocking the processes of viral infection, or for strengthening the natural defence of plants towards viruses.

As in the majority of host-parasite relations, plants have developed certain natural defence mechanisms against the viruses that infect them, some of which are starting to be discovered (Baulcombe, *Current Opinion in Plant Biology,* 2, 109 (1999); Kooter et al., *Trends in Plant Science* 4, 340 (1999), Waterhouse et al., *Trends in Plant Science* 4, 452 (1999). A defence mechanism mediated by RNA and directed towards the sequences of the pathogen has recently been detected in various viral infections in plants (Baulcombe, *Plant Cell* 8, 1833 (1996). This phenomenon was initially detected in transgenic plants constitutively resistant to viral infection, wherein there existed a correlation between resistance and low levels of expression in the cytoplasm of transgenes homologous to the sequences of viruses under study (Dougherty and Dawn Parks, *Current Opinion in Biology, 7,* 399 (1995); English *et al. Plant Cell* 8, 179 (1996). This mechanism, known as post-transcriptional genic silencing (hereinafter referred to as PTGS), is typically based on a sequence-specific degradation process of RNA, and seems to be a concrete manifestation of a more general phenomenon of natural defence in plants towards certain viruses, would become degraded by the effect of this resistance strategy (Al-Kaff *et al., Science* 279, 2113 (1998); Covey *et al., Nature* 386, 781 (1997); Ratcliff *et al., Science* 276, 1558 (1997); Ratcliff *et al., Plant Cell* 11, 1207 (1999). The defence mechanism would be induced in the plant by identifying some structural characteristic inherent to viral nucleic acids, such as might be the accumulation during the infection process of RNAs not normally present and therefore foreign to the plant cell. The formation of dsRNAs during viral replication constitutes one possible candidate, since the double-strand structure would suffice for being recognised by the cell as foreign RNA. The plant would then react, unleashing a signalling and degradation process at the cytoplasmic level analogous to that of PTGS, which would result in a progressive decrease in the quantity of virus in the plant during the infection process, giving rise to resistance to the viral infection (Covey *et al., Nature* 386, 781 (1997); Fire, *Trends in Genetics* 15, 358 (1999); Kooter *et al., Trends in Plant Science* 4, 340 (1990); Montgomery and Fire, *Trends in Genetics* 14, 255 (1998). Although the action mechanism of the phenomenon of genic silencing is not known, the discovery that viruses can both induce it and be its targets has led to the conclusion that genic silencing is a natural defence mechanism by which the plants recognise and fight foreign nucleic acids (Kasschau and Carrington *Cell* 95, 461 (1998); Smyth, *Current Biology* 9, R100 (1999). As a counterpart, viruses also defend themselves against this response from the plant by means of the expression of PTGS suppresser genes which are coded in its genomes, this counterattack strategy being widely distributed among plant viruses, both RNA and DNA (Voinnet *et al., Proc. Natl. Acad. Sci. USA*, 96, 14147 (1999).

The term pathogen-derived transgenic resistance (hereinafter referred to as PDTR) typically refers to the resistance obtained towards a certain pathogen, by means of using genetic engineering to introduce sequences of the genome of that pathogen into the plant genome (Sanford and Johnston, *J. Theoretical Biol.* 113, 395 (1985). Numerous gene sequences, deriving from many different plant viruses, have been introduced into a large variety of plant species, in order to produce transgenic plants protected against infection by these viruses. Many examples of PDTR against viruses in plants are attributed to a PTGS mechanism, a process, as stated above, mediated by RNA which identifies the sequences both of the viral RNA and of the transcribed mRNA of the transgene and degrades them in a specific way (Lindbo *et al., Plant Cell* 5, 1749 (1993); English *et al., Plant Cell* 8, 179 (1996); Covey *et al.*, *Nature* 386, 781 (1997); Tenllado and Diaz-Ruiz, *Transgenic Research* 8, 83 (1999); Vaucheret *et al., Plant J,* 16, 651 (1998); Sijen and Kooter BioEssays 22, 520 (2000): It has recently been proposed that the PTGS mechanism involves three different phases: initiation of the process, systemic propagation of a silencing signal and maintenance of the process (Palauqui and Vaucheret, *Proc. Natl. Acad. Sci. USA* 95, 9675 (1998); Ruiz et al. Plant Cell 10, 937 (1998). An important aspect of the second phase of this process is the capacity of the plant to send systemic signals to its tissues in such a way that the PRGS is propagated throughout the entire individual (Guo *et al., Molecular Plant-Microbe Interactions* 12, 103 (1999); Palanqui *et al. EMBO J.* 16, 4738 (1997); Voinnet and Baulcombe, Nature 389, 553 (1997). Although it has not yet been demonstrated, it is highly likely that the determining signal be a nucleic acid, and, for the cases that have been studied, the presence of small molecules of 25 nucleotides of antisense RNA, which could constitute or be components of the specific systemic signal (Hamilton and Baulcombe, Science 286, 950 (1999) has recently been confirmed. Another important aspect of the final phase is that a nuclear component with a similar sequence to the target mRNA seems to be absolutely necessary for the PTGS to perpetuate itself in time and space (Palauqui and Balzergue, *Curr. Biol.* 9, 59 (1999); Dalmay *et al., Plant* *Cell* 12, 369 (2000).

PTGS seems to be an adaptive strategy having widespread value in biology, since it is a phenomenon that is very similar and related to other processes that have been discovered recently in different taxonomic groups of animals and known as RNA interference (hereinafter referred to as RNAi). RNAi was described for the first time in the nematode *Caenorhabditis elegans* wherein the direct injection of dsRNA originated a sequence-specific PTGS (Fire *et al., Nature* 391, 806 (1998). Moreover, the same PTGS is obtained when the nematode is fed with bacteria which express dsRNA (Timmons and Fire, *Nature* 395, 854 (1998) or when they are produced by transcription of a transgene possessing an inverted and repeated sequence capable of forming dsRNA (Tavernarakis *et al., Nat. Genet.* 24, 180 (2000). Also, a similar phenomenon known as "quelling" has been described in the fungus *Neurospora crassa* (Cogoni and Macino, *Curr. Opin. Microbiol.* 2, 657 (1999): Subsequently, RNAi phenomena have been described in a varied range of different organisms (Fire, *Trends* Genet. 15, 358 (1999); Bass, *Cell* 101, 235 (2000). Bearing in mind that all these interference processes occur post-transcriptionally and produce the specific degradation of mRNA, it has been proposed that both RNAi and "quelling" and PTGS in plants, are related phenomena, which could play an important role in biology, protecting the genomes of organisms against foreign nucleic acids (Montgomery *et al., Proc. Natl. Acad. Sci. USA,* 95, 15502 (1998); Ngo *et al. Proc. Natl. Acad. Sci. USA* 95, 14687 (1998).

As occurs with RNAi in animals, PTGS can be very efficiently provoked in transgenic plants by the presence of a repeated and inverted sequence in the transcription region of the transgene (Hamilton et *al., Plant* J. 15, 737 (1998); Chuang and Meyerowitz, *Proc. Natl. Acad. Sci. USA* 97, 4985 (2000); Levin *et al., Plant Mol. Biol.* 44, 759 (2000). In addition, it has been seen that tobacco plants transformed with a construction that transcribes RNA capable of forming dsRNA produces immunity towards the virus or genic silencing, with almost 100% efficacy, depending on whether the target is a virus or an endogenous gene (Waterhouse *et al*. *Proc. Natl. Acad. Sci. USA* 95, 13959 (1998); Smith *et al*. *Nature* 407, 319 (2000). Therefore, having collectively analysed these phenomena, there exists strong evidence indicating that dsRNA molecules act as inductors of the first phase of the PTGS process, in both the plant and the animal kingdoms. Nevertheless, there still does not exist direct proof of the *in vivo* formation of dsRNA in transgenic plants.

We have discovered that the multiplication of viruses in plants can be interfered with in a specific way, by means of the direct application of homologous dsRNA, which extends the previously discoveries on RNAi in animals, to plants. More concretely, our discovery shows that the direct application of exogenous dsRNA in non-transgenic plants can act as a resistance inductor towards viruses. This interference mediated by dsRNA in plants has characteristics in common with RNAi in animals: the interference is sequence-specific, it is induced by dsRNA and not by ssRNA and it requires a minimum size of dsRNA.

The direct use of exogenous dsRNA molecules in plants for blocking the multiplication of viruses in cells, which is the object of the present invention, has not yet been described in the scientific literature.

### Brief description of the invention

A first aspect of this invention is the cloning of any fragment of the genome of any virus whose multiplication in the cells of the plant it is wished to block.

A second aspect of this invention is the insertion of the cloned fragment into a transcription vector in order to synthesise fragments of ssRNA of both polarities (sense/antisense) or complementary fragments and to mix the products of both transcriptions in order to obtain the molecules of dsRNA of identical sequence to that of the genome of the virus, or very similar to that of other viruses, whose multiplication it is wished to prevent.

A third aspect of this invention is the application of dsRNA molecules in some tissue of the plants (e.g., leaves, roots, etc.) in order to interfere with the infection by the corresponding virus whose genome has a similar sequence to the dsRNA.

The novelty of this invention lies in the fact that it is the first time that the direct application of dsRNA is described in plants for interfering with and silencing in a specific way transitory expression genes, such as virus genes, with the consequent protection of the plants against the corresponding viral diseases, without having to employ viral vectors or resort to procedures for genetic transformation of plant cells.

This invention could be used for reducing the serious economic losses caused by viruses in many species of agricultural interest around the world, and also, indirectly, for avoiding the abusive use of pesticides employed for controlling their insect vectors, thus leading to protection of the environment and the avoidance of ecological risks.

By means of this invention, it should be possible to also silence in plants the expression of both endogenous genes and transgenes in a specific way, by means of the direct application of dsRNA sharing sequence-similarity with the target gene or transgene.

### Detailed description of the invention

The object of this invention is the direct application of molecules of exogenous dsRNA in any tissue of any plant, in order to interfere in a specific way with the multiplication in the plant cells of the corresponding virus having sequence-similarity with the dsRNA.

The description of this invention starts from at least three plant viruses belonging to different taxonomic groups, representing extreme examples in the evolution of RNA viruses in plants, such as pepper mitigated mottled virus (hereinafter referred to as PMMoV), tobacco engraved virus hereinafter referred to as TEV) and alfalfa mosaic virus (AMV). The three viruses have RNA genomes for which their complete sequence is known (Alonso *et al., J. Gen.* Virol. 72, 2875 (1991); Allison *et al., Virology* 154, 9 (1986); Neelman *et al., Virology* 181, 687 (1991) and they furthermore possess different replication and genetic expression strategies and have been employed here as markers in order to confirm the widespread applicability of the procedure. PMMoV (Alonso *et al., J. Gen. Virol.* 72, 2875 (1991) belongs to the genus *Tobamovirus*, a group of viruses with a genome formulation made up of a single molecule of messenger sense ssRNA. AMV (Neelman *et al., Virology* 181, 687 (1991) belongs to the genus *Alfamovirus,* within the family *Bromoviridae*, a group of viruses with a genome organisation made up of three molecules of messenger sense ssRNA. Both viruses are to be found within the superfamily of sindbisviruses. TEV (Dolja et *al., Proc. Natl. Acad. Sci. USA* 89, 10208 (1992) belongs to the genus *Potyvirus,* within the family *Potyviridae,* a group of viruses with a genome organisation made up of a single molecule of messenger sense ssRNA, whose genome organisation is similar to that of the superfamily of picornaviruses.

First of all, at least five fragments of different sizes of certain known genes of these viruses were subcloned in commercial plasmidic vectors, these fragments being: (1) a fragment of 977 base pairs (hereinafter referred to as bp) coming from the 54K region of the PMMoV polymerase gene, (2) a fragment of 315 bp coming from the same 54K region of the PMMoV polymerase gene, (3) a fragment of 596 bp coming from the region of the 30K movement gene of PMMoV, (4) a fragment of 1124 bp coming from the region of the movement protein / coverage protein genes of AMV RNA 3, and (5) a fragment of 1483 bp coming from the region of the HC-Pro gene of TEV. The cloning was carried out starting from complete clones of complementary DNA (hereinafter referred to as cDNA) of these viruses, using conventional procedures (Sambrook *et al., Molecular cloning:* A laboratory *manual,* 2 edn. Cold Spring Harbor, NY, Cold Spring Harbor Laboratory (1989), which can be easily carried out by those persons who are expert in the subject. This cloning can also be done by means of the known technique of reverse transcription followed by polymerase chain reaction (hereinafter referred to RT-PCR), starting from extracts of plant RNA infected with the respective viruses, using appropriate synthetic oligonucleotides and commercial vectors available on the market.

After that, the previously cloned fragments were inserted into commercial transcription vectors and transcribed in vitro using the promoters of polymerase RNA of the phages T3 and T7, using conventional procedures well known to persons familiar with the subject. A fragment of 1605 bp corresponding to the complete 54K gene of PMMoV was also inserted in the sense or antisense orientation in a binary expression vector in plants, between a high expression promoter, such as the promoter 35S of cauliflower mosaic virus (hereinafter referred to as CaMV) and the transcriptional terminator of gene 7 of TL-DNA, and the corresponding constructions were introduced into the strain LBA 4404 of the bacterium *Agrobacterium tumefaciens*, using known procedures of direct transformation (An *et al*. Binary vectors. A3: 1-19 in: Plant Molecular Biology manual S.B. Gelvin and R.A. Schilperrot, eds., Kluwer Academic Publishers, Dordrecht (1988), widely used by persons expert in the subject. Also obtained, by means of the polymerase chain reaction (hereinafter referred to PCR), were molecules of cDNA corresponding to the same region of 977 bp of the 54K gene of PMMoV and, therefore of the same sequence as RNA products synthesised in vitro in the transcription reaction.

Both the molecules of sense and antisense ssRNA obtained from the in vitro transcription, and the molecules of dsRNA obtained from the mixing and hybridisation of the sense and antisense transcription products, or the molecules of cDNA obtained by PCR, were then directly applied on the leaves of hosts, such as plants of *Nicotiana benthamiana,* preferably by means of mechanical inoculation, together with the corresponding homologous viral inoculum, in the form of purified virus particles or as infective RNA. Also applied to the leaves of N. benthamiana, in a different experimental approach, was a mixture of cultures of A. *tumefaciens*, transformed with sense and antisense constructions of the complete 54K gene of PMMoV, by means of the known procedure of agroinfiltration (Vaucheret, *C. R. Acad. Sci. III* 317, 310 (1994), followed by inoculation in the same infiltrated leaves with the viral inoculum of PMMoV mentioned previously.

The inoculation of each of the different viruses in plants of *N. benthamiana* produces a systemic infection with symptoms preferably of mosaic, around seven days past inoculation (hereinafter referred to as dpi). When the different molecules of dsRNAs that are obtained, corresponding to the different viruses and coming from the mixing and hybridisation of the sense and antisense transcription products, were inoculated jointly with the homologous infective viral inoculum, into leaves of N. *benthamiana*, in no case did symptoms of viral infection appear, nor were viral RNAs detected, in the inoculated leaves or in the systemic leaves, between 7-10 dpi. Only in one case did the plants suffer a delay of 1-3 weeks in the appearance of the viral symptoms. On the contrary, in the rest of the plants coinoculated with homologous ssRNA of both polarities, homologous cDNA, or non-homologous viral dsRNA, together with the corresponding viral inoculum, viral symptoms always appeared and no reduction was detected in the accumulation of virus or viral RNAs in comparison with control plants, inoculated with just the viral inoculum in the transcription buffer.

The same result was produced by at least one dsRNA application procedure different from that of mechanical inoculation, when the leaves of *N. benthamiana* were treated by means of agroinfiltration, with the mixture of *A. tumefaciens* transformed with the sense and antisense constructions, whose transcription products have to form in vivo molecules of dsRNA, and later on the same leaves were inoculated with the homologous virus up to at least four days past the agroinfiltration treatment with the mixture of A. tumefaciens. The systemic leaves of the agroinfiltrated plants did not present any viral symptoms, nor were the corresponding viral RNAs detected in the infiltrated leaves nor in the systemic ones. On the contrary, in the rest of the plants agroinfiltrated with homologous ssRNA of one or another polarity, together with the later viral inoculum, viral symptoms always appeared and no reduction was detected in the accumulation of virus or viral RNAs in comparison with control plants, agroinfiltrated with *A. tumefaciens* transformed solely with the vector without the insert.

These results discard any possible *in* vitro interaction between the molecules of dsRNA and the viral inoculum in the above coinoculation experiment as the cause of the interference responsible for the protection effect of the plants towards viral infection. Similarly, the results of the specificity of the sequence, such as the structure of the dsRNA molecules, discard any possible device due to the inoculation procedure and demonstrate that only the dsRNA molecules, either directly applied on the surface of the leaf or by means of agroinfiltration, can activate the PTGS mechanism, impeding the replication of the virus with which they have sequence similarity, at least in treated leaves, and that the procedure can therefore be applied for defending the plants against viral infection.

In the same way, similar results were obtained using at least two non-systemic hosts, such as tobacco plants *(Nicotiana tabacum* cv. "Xanthi nc") or pepper plants *(Capsicum chinense*), where PMMoV only produces localised infection with symptoms of local lesions. When the different molecules of dsRNA, ssRNA of both polarities, cRNA homologous with the 54K gene region of PMMoV, and also dsRNA non-homologous with this virus, were coinoculated in half-leaves of the non-systemic hosts, with the virus being inoculated only in the opposite half-leaf, the formation of local lesions on the leaf surface was completely blocked only in the case of coinoculation with dsRNA, where no lesion at all occurred. In the rest of the cited combinations and in the opposite halves inoculated with an equivalent quantity of the virus, more than 50 local lesions always occurred.

These results also show that only the molecules of dsRNA can activate the PTGS mechanism, impeding in a specific way the formation of local lesions in at least two species of plants belonging to different genera and that, therefore, the procedure has general validity and could be applied for defending any plant against viral infections.

The invention described above is widely applicable to any other virus, known or yet to be known, possessing RNA as genetic material or wherein RNA is originated in any phase of its infective cycle, as will be recognised by those familiar with the art of virology.

The invention described above can also be used with any plant (angiosperm or gymnosperm, monocotyledon or dicotyledon) in which dsRNA can be applied and in which it is wished to interfere with the infection of any virus having the aforementioned characteristics, and liable to being infected by these viruses, in other words, in which the virus can replicate itself in the inoculated cells.

The invention described above can be used with any available method of direct introduction of dsRNA inside the plant cells, widely used by persons expert in the field, such as mechanical inoculation, infiltration or agroinfiltration, injection, electroporation or bombarding with microparticles, in the case of not many plants, or by vaporisation or fumigation procedures in the case of a large number of plants or crops, etc. The most appropriate place of introduction of dsRNA into the plant is, without excluding others, the tissue of the upper face of the leaf.

The invention described above shows that it is possible to interfere with viral infections in a specific way and that a PTGS of at least transitory expression genes (e.g., viral genes) can be induced in plants, by means of the direct application of dsRNA molecules of homologous sequence and therefore, the novelty of this invention is the direct application of any exogenous molecules of dsRNA into the tissue of any plant in order to protect plants against viral infections.

The invention described previously, concerning the direct application of dsRNA into plant cells by means of any procedure, is different from other strategies based on the expression in transgenic plants of RNAs with the capacity to form molecules of dsRNAs, such as for example the protection thus obtained by means of PTGS against potato virus Y (PVY) (Waterhouse *et al., Proc. Natl. Acad. Sci. USA*, 95, 13959 (1998); Smith *et al., Nature* 407, 319 (2000), and supports the idea of other researchers on the involvement of viral replicative intermediary dsRNAs as efficient indicators of PTGS in natural virus infections (Ratcliff, *et al., Science,* 276, 1558 (1997); Ratcliff, *et al., Plant Cell,* 11, 1207 (1999).

Moreover, it can be predicted that the invention described above can also be used for specifically silencing the expression of any endogenous gene or transgene of the plant, which represents an alternative to the genetic transformation procedures of plants with constructions capable of expressing dsRNA, with a view to specifically interfering with the expression of endogenous genes in plants (Chuang and Meyerowitz, *Proc. Natl. Acad. Sci. USA*, 97, 4985 (2000); Levin *et al., Plant Mol. Biol.* 44, 759 (2000).

The Examples described below serve to illustrate the present invention in greater detail, though without limitation.

### EXAMPLE 1

### Synthesis of RNAs and direct application by means of inoculation of plants

For the production of dsRNA molecules, strands of sense and antisense ssRNA were synthesised in vitro, starting from the corresponding plasmid of cDNA, using the promoters of the polymerase RNA of phages T3 and T7 (T3/T7 transcription kit, Roche), by described procedures (Sambrook *et al., Molecular cloning: A laboratory manual,* 2 edn. Cold Spring Harbor, NY, Cold Spring Harbor Laboratory (1989). The strands of synthesised ssRNA (2.5 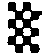 M) were mixed and hybridised by warming at 95°C for three minutes in a sodium phosphate buffer 25 mM pH 7, and subsequent cooling to 37°C for 30 minutes. The formation of dsRNA molecules was confirmed by means of analysing the change of their mobility in agar gel in comparison with the mobility of each of the sense and antisense ssRNA molecules, and also due to their resistance to RNAsa A (Roche) under conditions of high salt concentration.

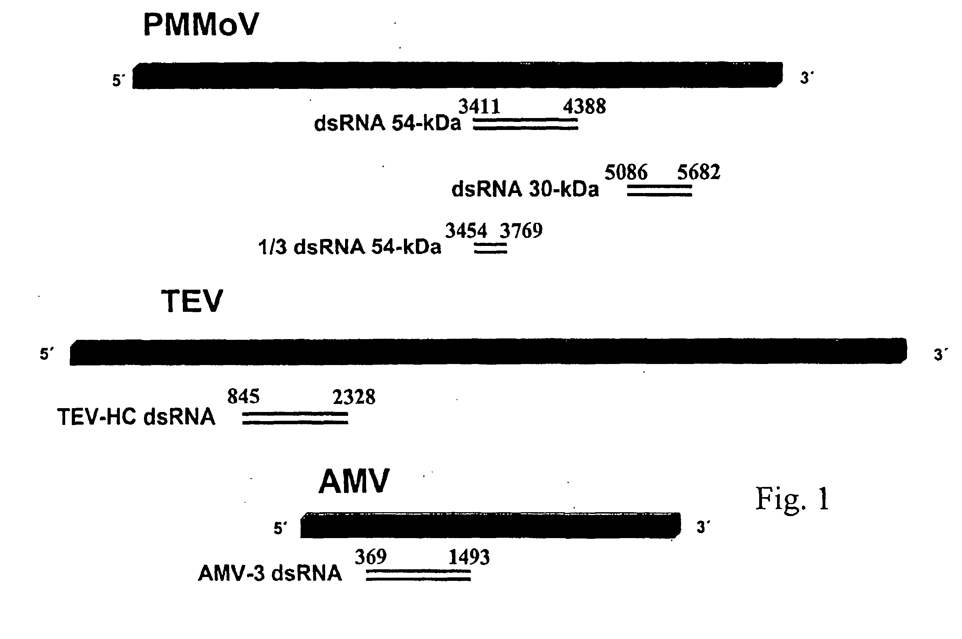

For the production of molecules of dsRNA derived from PMMoV, three fragments were cloned starting from a complete clone of PMMoV cDNA, corresponding to the positions 3411-4388, 5086-5682 and 3454-3769, in the PMMoV sequence (Alonso *et al., J. Gen.* Virol. 72, 2875 (1991) (Fig. 1) in the vector pT3T7 (Roche), which, following the corresponding transcription and hybridisation, gave rise to dsRNA fragments of 977-bp (hereinafter referred to as 54-kDa dsRNA), 596-bp (hereinafter referred to as 30-kDa dsRNA) and 315-bp (hereinafter referred to as 1/3 54-kDa dsRNA), respectively.

For the production of molecules of dsRNA derived from TEV, a fragment corresponding to the positions 845-2328 in the TEV sequence was cloned (Dolja et *al., Proc. Natl. Acad. Sci. USA* 89, 10208 (1992) (Fig. 1) in the vector pBluescript SK-(New England Biolabs) which, following the corresponding transcription and hybridisation, gave rise to the dsRNA fragment of 1483-bp (hereinafter referred to as TEV-HC dsRNA).

For the production of molecules of dsRNA derived from AMV, a fragment corresponding to the positions 349-1493 in the sequence of AMV RNA 3 was cloned (Neelman *et al., Virology* 181, 687 (1991) (Fig. 1) in the vector pBluescript SK-(New England Biolabs) which, following the corresponding transcription and hybridisation, gave rise to the dsRNA fragment of 1124-bp (hereinafter referred to as AMV-3 dsRNA).

All the plasmids were linearised with the corresponding commercially available restriction enzymes (Roche, New England Biolabs) and were used as a mould for *in vitro* transcription reactions and the consequent generation of sense and antisense ssRNA molecules.

For the production of molecules of PMMoV cDNA, the starting point was an exact copy of the 54-kDa gene of PMMoV (positions 3499-4908), which gave rise to the corresponding molecules of cDNA (hereinafter referred to as 54-kDa cDNA) by means of PCR (thermocyclator and Taq 1 DNA polymerase, Perking-Elmer), using the appropriate primers described previously (Tenllado *et al.*, *Virology* 211, 170 (1995).

For the interference experiments with PMMoV infection, a standard inoculum of 10 µg/ml of purified virus was used (Alonso et *al., J. Gen. Virol.* 72, 2875 (1991). For the interference experiments with TEV infection, the plasmid pTEV-7D (kindly provided by Dr. J.C. Carrington, Washington State University), which contains a complete cDNA clone of TEV, was linearised and transcribed *in vitro* with the polymerase SP6 RNA, as described by Dolja *et al., Proc. Natl. Acad. Sci. USA* 89, 10208 (1992). For the interference experiments with AMV infection, the transcription with T7 RNA polymerase of complete clones of RNA 1 (pUT17A), RNA 2 (pUT27A) and RNA 3 (pAL3) of AMV (kindly provided by Dr. J.F. Bol, Leiden University), was carried out as described previously (Neelman *et al., Virology* 254, 324 (1999). The inoculation mixtures were produced by means of the addition of 5 µl of dsRNA of each virus (approximate concentrations of 0.8 µg/µl, estimated by staining with ethidium bromide with markers of known weight) at an equivalent volume of purified virus (PMMoV) or at 10 µl of viral transcripts (TEV and AMV). In the case of AMV, 10 µg of protein from the AMV capsid were added to the inoculation mixture.

The inoculation of the plants was done using two fully open leaves in at least two plants per test, by means of gentle pressure on the leaf surface with the inoculum, using commercial carborundum as abrasive (Matthews, Plant Virology, Third Edition (San Diego, *Academic Press, Inc.)* (1991). In the comparisons of the effect on the viral infection of sense and antisense ssRNA molecules, dsRNA and of cDNA, equivalent molar concentrations were used. The inoculated plants were kept in culture chambers under standard growth conditions, with the development of symptoms of viral infection in systemic hosts being checked during the time that their life-cycle lasted for. In hosts with local lesion, the inoculated leaves were observed during at least 5 dpi.

### EXAMPLE 2

### Analysis of viral RNAs in plants

For the analysis of viral RNAs in plants, extracts of total RNA were obtained starting from inoculated leaves, between 6 and 10 dpi, and from systemic leaves, between 6 and 21 dpi, according to the method described by Logemann at el. Anal. *Biochem*. 163, 16 (1987). The samples of total RNA (between 1 and 5 µg) were separated by means of electrophoresis in 1-1.2% agar-formaldehyde gels and were transferred to Hybond-N membranes (Roche). Stainings were carried out with ethidium bromide in agar gels prior to their transference to Hybond-N membranes, in order to confirm the integrity of the RNA and to ensure a similar charge of quantities in each sample. Northern blot type hybridisation was carried out using RNA probes marked with digoxigenin (Roche) as described by Neelman and Bol (1999).

Specific RNA probes were used to detect the RNAs of the different viruses. PMMoV RNA was detected with a complementary probe to the nucleotides 3411-4388 of PMMoV, which were transcribed starting from the clone pT3T7/54-kDa (Tenllado *et al., Virology,* 211, 170 (1995). TEV RNA was detected with a complementary probe to the nucleotides 845-2328 of TEV, which were transcribed starting from the clone pBluescript SK-/HC. AMV RNAs 3 and 4 were detected with a complementary probe to the nucleotides 369-1493 of the RNA 3 of AMV, transcribed starting from the clone pBluescript SK-/AMV-3.

### EXAMPLE 3

### Transitory expression of dsRNA in plants by means of Agrobacterium tumefaciens

For the transitory expression of dsRNA in plants, the region of PMMoV RNA which codes the 54-kDa protein and flanking sequences (nucleotides 3411-5016) were inserted in the sense or antisense orientation between the promoter 35S of CaMV and the transcriptional terminator of gene 7 of TL-DNA, in the binary vector pGSJ780A (Plant Genetic System), as described in Tenllado et *al., Virology* 211, 170 (1995). These constructions were introduced into the strain LBA 4404 of *A*. *tumefaciens* by means of direct transformation (An *et al.*, Binary vectors. A3: 1-19 in: Plant Molecular Biology Manual. S.B. Gelvin and R.A. Schilperrot, eds. Kluwer Academic Publishers, Dordrecht (1988). The recombinant A. *tumefaciens* were grown for 16 hours at 28°C in tubes containing 10 ml of LB medium, supplemented with 50 µg/ml of rifampicin and 40 µg/ml of streptomycin. The cells were precipitated and resuspended at a final concentration of 0.5 OD₆₀₀ in a solution containing 10 mM of MgCl₂, 10 mM Mes (pH 5.6) and 150 µM of acetosyringon. The cultures were incubated at 28°C for 2-3 hours before proceeding to infiltration of two leaves per plant, by means of a 1 ml non-needle syringe, the entire plant then being covered with a transparent plastic bag for 2 days. In coinfiltration experiments of *Agrobacterium* cultures with 54-kDa sense and antisense constructions, equal volumes of both cultures were mixed prior to their infiltration.

### EXAMPLE 4

### Direct application of dsRNA molecules derived from PMMoV in two local hosts

Experiments were conducted in order to demonstrate that direct application by means of mechanical inoculation of a molecule of dsRNA derived from PMMoV, together with the viral inoculum, can interfere in a specific way with the viral infection in a local host. To achieve this, molecules of ssRNA corresponding to the 54K region of the gene of PMMoV replicase were transcribed in vitro and in the sense and antisense orientation, and the transcription products were mixed and hybridised to produce 54-kDa dsRNA, as described previously. Both the molecules of dsRNA and those of sense and antisense ssRNA were analysed in order to determine their capacity to interfere with the development of local lesions produced by PMMoV, in the hypersensitive host *Nicotiana tabacum* cv. "Xanthi nc". To do this, half-leaves were inoculated with the virus alone and the opposite half-leaf was inoculated with the virus with the transcription buffer only, or with sense ssRNA, antisense ssRNA and 54-kDa dsRNA, at final concentrations of 0.62 µM.

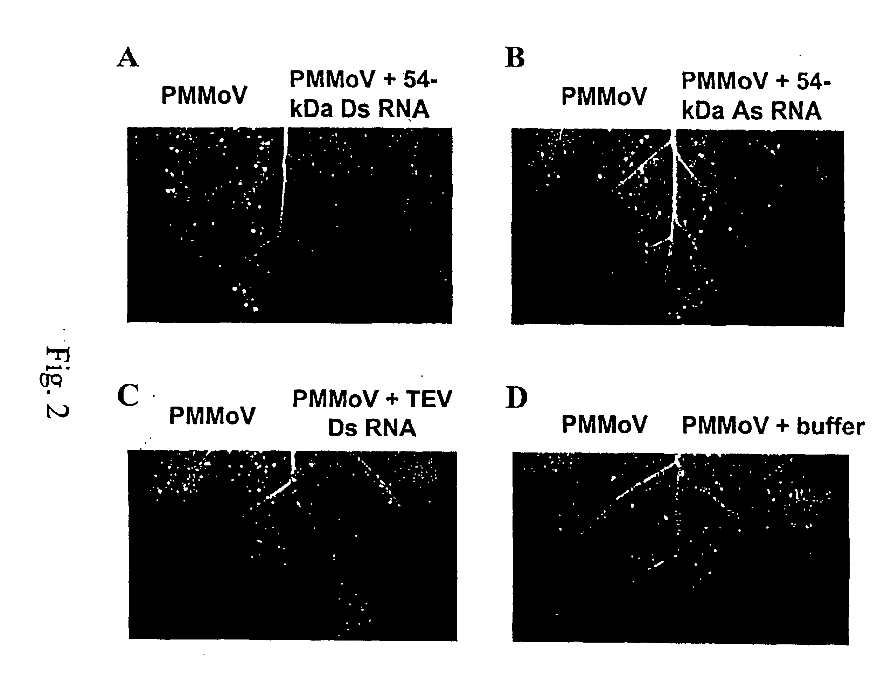

Figure 2 shows a representative example of different experiments performed with RNAs produced in different transcription reactions. The infectivity of PMMoV, determined by the formation of local lesions on the leaf surface, was completely blocked in the case of coinoculation with 54-kDa dsRNA (Fig. 2A), where no lesion took place, while on the opposite half of the leaf, inoculated with an equivalent quantity of the virus alone, more than 50 local lesions were produced. Nor was the formation of lesions blocked in cases of coinoculation of the virus with either of the sense or antisense ssRNAs (Fig. 2B and not shown). Moreover, the coinoculation of PMMoV with a dsRNA of viral origin, though not related to PMMoV but instead derived from TEV (TEV-HC dsRNA), also did not produce any effect on the infection by the virus and did not block the formation of local lesions (Fig. 2C). Similar experiments to those described above, but using the hypersensitive host *Capsicum chinense* instead of *N. tabacum,* were also conducted, with the same result of complete blocking of the infection only in the case of coinoculation with homologous dsRNA.

Therefore, infection by PMMoV can be blocked specifically with at least the 54-kDa dsRNA molecule and in at least two local hosts belonging to different plant genera.

### EXAMPLE 5

### Direct application of different molecules of dsRNA derived from PMMoV in a systemic host

Experiments were also conducted in order to demonstrate the capacity of the 54-kDa dsRNA molecule to interfere with PMMoV infection in a systemic host. To do this, plants of *N. benthamiana* were used, which were inoculated with mixtures of PMMoV plus each of the transcription products derived from PMMoV and described above. Also included in the experiments were molecules of cDNA obtained by means of PCR and corresponding to the same 54K region of the virus as the RNA products synthesised in vitro in the transcription reaction (54-kDa cDNA), as described above.

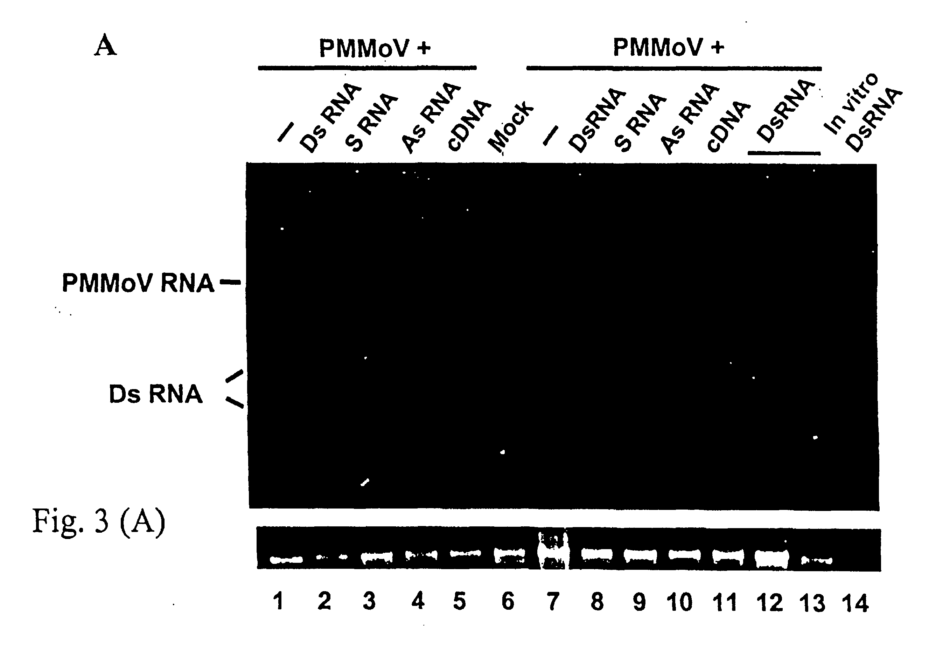

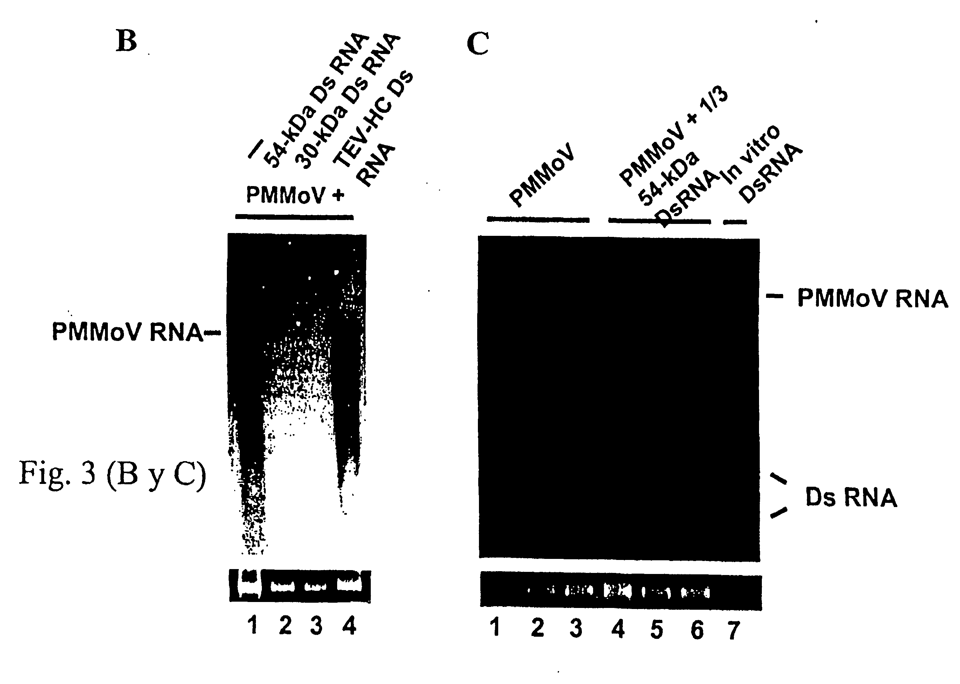

At 7 dpi, all the plants inoculated with mixtures of PMMoV plus the sense or antisense ssRNA molecules or the cDNA molecules were infected and showed systemic symptoms, except for plants coinoculated with the mixture of PMMoV plus 54-kDa dsRNA, which were protected against viral infection and showed no symptoms of disease. Consistent with these results, Northern blot analysis of the total RNAs extracted from the inoculated and systemic leaves at 7 dpi showed that the plants coinoculated in the presence of sense or antisense ssRNAs, or in the presence of cDNA molecules homologous to the virus, accumulated positive sense RNA of PMMoV at levels comparable with those shown by the control plants (Fig. 3A). Nevertheless, the multiplication of PMMoV was blocked in the inoculated leaves when the molecule used in the coinoculation was 54-kDa dsRNA, and neither did the virus become accumulated at detectable levels in the upper leaves of those plants. Nor was the accumulation of negative sense RNA of PMMoV detected in those samples.

In order to corroborate the interference mediated by 54-kDa dsRNA in PMMoV infection, more than ten independent tests were conducted with 22 plants. In the majority of cases, the plants stayed free of symptoms until their life-cycle was completed, without accumulating viral RNA at detectable levels. Nevertheless, in some cases, the virus overcame the protection conferred by 54-kDa dsRNA and plants showed signs of disease, though always with a delay of between 1 and 3 weeks compared to the control plants. The PMMoV RNA became accumulated in the upper leaves of these plants at moderate levels.

In all these experiments, and repeatedly so, bands of hybridisation corresponding to partially denaturalised 54-kDa dsRNA molecules were observed in the Northern blot analysis of preparations of total RNA, obtained starting from the leaves of plants inoculated with virus plus 54-kDa dsRNA, or inoculated only with 54-kDa dsRNA, as was confirmed when comparing these hybridisation bands with the behaviour of the 54-kDa dsRNA preparation used in the inoculum and likewise analysed. Numerous experiments were conducted in order to determine the origin of those hybridisation bands corresponding to 54-kDa dsRNA molecules. The analysis of the degradation kinetics of those molecules confirmed that the dsRNA used in the inoculum remains relatively stable and persists in the inoculated leave at detectable levels, at least up to 7 dpi.

The interference in PMMoV infection shown by 54-kDa dsRNA which, as has been described above, corresponds to the 54-kDa region of the gene that codes the PMMoV replicase, could reflect some kind of inhibiting effect of this sequence in particular on infection by the virus. Therefore, a determination was made of whether other dsRNA molecules derived from a different region of the PMMoV genome could also specifically block infection by PMMoV, when introduced into the plant simultaneously with the virus. In order to conduct this experiment, a dsRNA was obtained corresponding to a fragment of 595 bp of the gene 30-kDa of the PMMoV movement protein (30-kDa dsRNA), as described previously. The effect of this 30-kDa dsRNA on the viral infection was compared with the known effect occasioned by 54-kDa dsRNA or, also, with the effect occasioned by a dsRNA of viral origin but not homologous with PMMoV, such as that obtained from the fragment of 1483 bp corresponding to the larger part of the HC gene of TEV (TEV-HC dsRNA), as described previously. As with 54-kDa dsRNA, the presence of 30-kDa dsRNA in the inoculum blocked the expression of viral symptoms in N. benthamiana, during times in which the control plants showed symptoms of infection. Parallel with this, no accumulation of viral RNA was detected in the RNA extracted from those plants on the basis of the tissue corresponding to the upper leaves (Fig. 3B). Nevertheless, inoculation of PMMoV together with TEV-HC dsRNA, a non-homologous dsRNA, had no effect on the expression of viral symptoms and the PMMoV RNA accumulated in the upper leaves of those plants at the same level as in the control plants. This same result - the incapacity of the non-homologous dsRNA molecule to interfere with PMMoV infection - has also been observed using different fragments of dsRNA of non-viral origin and with variable lengths.

Therefore, interference with PMMoV infection takes place when using any molecule of dsRNA, provided it shares sequence similarity with the virus.

### EXAMPLE 6

### Direct application of dsRNA molecules derived from different viruses

Experiments were conducted in order to determine whether the use of dsRNA molecules could constitute a strategy of general application for protecting plants against infection by viruses other than PMMoV. For this, viruses were used having no relation with the tobamovirus genus such as TEV, which belongs to the family *Potyviridae*, and AMV, which forms part of the family *Bromoviridae*, and the effect was studied of different dsRNAs derived from this virus on infection by their corresponding viruses in a systemic host.

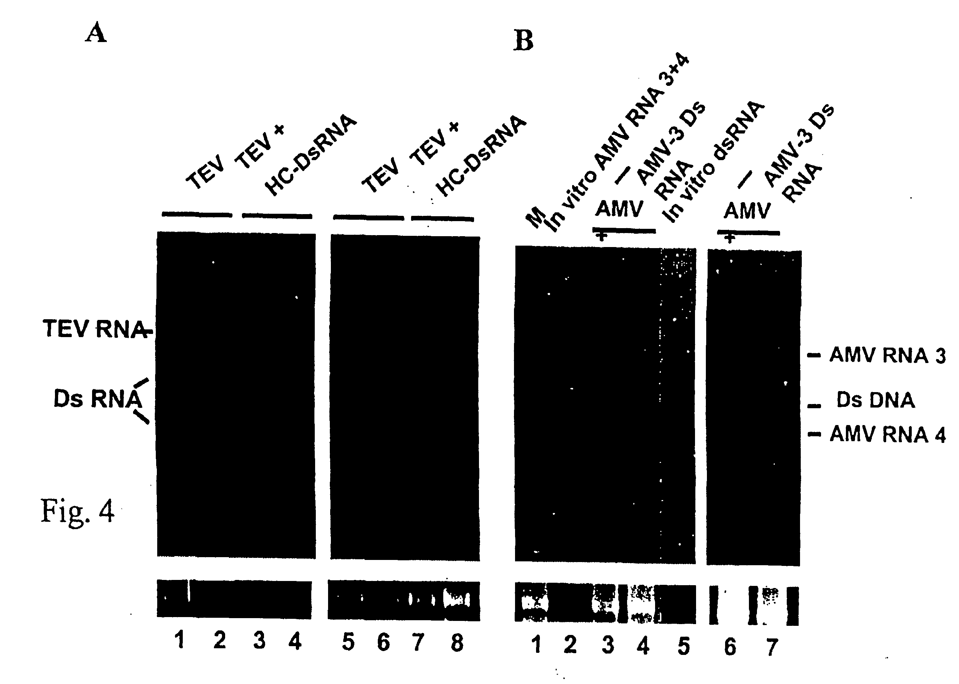

Plants of N. tabacum were used, which were inoculated solely with SP6 infective transcripts corresponding to a cDNA clone of TEV, or with a mixture containing the above RNA transcripts of TEV plus the dsRNA homologue TEV-HC dsRNA. Two weeks after inoculation, the plants inoculated with the mixture showed no signs of either localised lesions on the leaf nor systemic symptoms in the upper leaves, while plants inoculated solely with TEV showed symptoms of disease at 6 dpi. Figure 4A shows a Northern blot analysis of the total RNAs extracted from two plants by treatment at 6 dpi. The TEV RNA was accumulated both in the inoculated leaves and in the systemic leaves of the control plants. Nevertheless, the levels of viral RNA were below the detection limit in plants inoculated with a mixture of virus and homologous dsRNA. As described previously, in this case too hybridisation bands of variable intensity were observed, corresponding to TEV-HC dsRNA in the total RNA extracted from the leaves inoculated with virus plus dsRNA.

In a similar way, plants of *N. benthamiana* were inoculated with, on the one hand, a mixture of T7 transcripts of the genome RNAs 1, 2 and 3 of AMV and the capsid protein of AMV, since one of the distinctive characteristics of alfamoviruses is that a mixture of three genome RNAs of the virus is not infective in plants unless the capsid protein of the virus is added to the inoculum (Bol 1999), and on the other hand, they were inoculated with the aforementioned mixture plus a homologous RNA including a fragment of 1124 nucleotides of RNA 3 of AMV (AMV-3 dsRNA). Figure 4B shows a Northern blot analysis of total RNAs extracted from inoculated or systemic leaves of those plants, using a probe which recognises genome RNA 3 and subgenome RNA 4 of AMV. Used as controls in the gel were the RNAs 3 and 4 of AMV transcribed *in vitro* and the AMV-3 dsRNA used in the inoculum. At 6 dpi, plants inoculated with the mixture of the RNAs of AMV plus AMV-3 dsRNA showed no symptoms of disease, while plants inoculated solely with the RNAs of AMV were susceptible to viral infection, showing systemic symptoms. In line with the symptoms observed, no accumulation of the RNAs of AMV was detected in plants inoculated with the mixture of genome RNAs of AMV plus AMV-3 dsRNA but, on the contrary, in plants inoculated solely with the RNAs of AMV, the RNAs 3 and 4 of the virus became accumulated both in the tissue of inoculated leaves and in the tissue of leaves in the upper part of the plant.

Therefore, it is possible to interfere in a specific way with the infection of any virus by means of applying any molecule of dsRNA, provided it shares sequence similarity with the virus.

### EXAMPLE 7

### Specific interference mediated by size-dependent dsRNA molecules

Experiments were conducted in order to determine whether the capacity to interfere with viral infections in plants in a specific manner, by means of applying molecules of dsRNA, is dependent upon the size of the dsRNA molecule since, as has been demonstrated previously, both the 54-kDa dsRNA and the 30-kDa dsRNA molecule (of 977 bp and 596 bp, respectively) were efficacious in protecting the plants against infection by PMMoV. To do this, plants of *N. benthamiana* were used, which were inoculated with the mixture of PMMoV plus a smaller dsRNA, of 315 dp, derived from PMMoV and approximately corresponding to one third of the length of 54-kDa dsRNA (1/3 54-kDa dsRNA), obtained as described above. Unlike in cases of 54-kDa dsRNA and 30-kDa dsRNA, described previously, the coinoculation of PMMoV plus the molecule 1/3 54-kDa dsRNA only had a marginal effect on infection by the virus. The appearance of viral symptoms in these plants was delayed by 1-2 days compared with signs of symptoms in plants inoculated solely with virus, and the viral RNA became accumulated at levels similar to those of the control (Fig. 3C).

Therefore, the capacity of dsRNA to interfere specifically with the viral infection is dependent on the length of the dsRNA molecule used.

### EXAMPLE 8

### Specific interference mediated by dsRNA molecules inside the plant

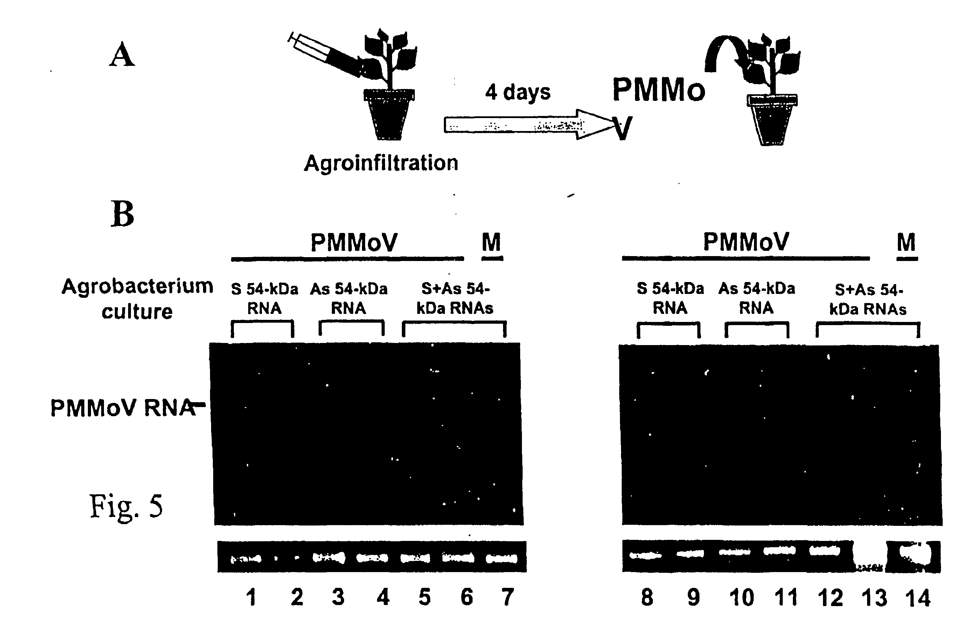

Experiments were also conducted in order to determine whether coinoculation with dsRNA molecules together with viral particles or infective viral RNA, in order to prevent infection by different plant viruses in a specific manner, is due to the existence of any kind of inhibiting interaction that takes place before the virus or the viral RNA penetrates the cell. To do this, a transitory expression test was conducted, mediated by *Agrobacterium* (agroinfiltration, Vaucheret, *C. R. Acad. Sci. III* 317, 310 (1994), with the aim of testing whether the molecules of dsRNA inhibit viral infection when directly expressed inside the plant cell. First, the complete 54-kDa region and flanking sequences of PMMoV (nucleotides 3411-5016) were cloned, in the sense or antisense orientation, under the control of the constitutive promoter 35S of CaMV in a binary vector, as described previously. Then, cultures of *A. tumefaciens* carriers of the vectors that express sense or antisense 54-kDa RNA were mixed in a proportion of 1:1 and the mixture was coinfiltrated in leaves of N. benthamiana. As control, plants with carrier cultures of just sense or antisense 54-kDa ssRNA were agroinfiltrated separately. Four days after agroinfiltration, the plants were inoculated with PMMoV directly on the infiltrated leaves. In three independent experiments, all the plants transitorily expressing either sense 54-kDa RNA or antisense 54-kDa RNA showed signs of disease in the upper leaves at 10 dpi, while plants agroinfiltrated with vectors expressing the sense and antisense 54-kDa RNA mixture showed no symptoms, or their appearance was delayed by between 1 and 3 weeks compared to the controls. Figure 5 shows a Northern blot analysis of the accumulation of PMMoV RNA in preparations of total RNA extracted from two individuals by treatment at 15 dpi. In the inoculated leaves of the plants infiltrated with the mixture of sense and antisense strands of ssRNA, which have the capacity for ringing with one anther and forming a dsRNA structure inside the cell, the PMMoV RNA either did not accumulate or did so at very low levels, while PMMoV RNA was never detected in the upper leaves of those plants. On the contrary, neither sense 54-kDa RNA nor antisense 54-kDa RNA, expressed by *Agrobacterium,* interfered with the accumulation of PMMoV, both in the inoculated leaves and in the systemic leaves of the control plants.

Therefore, specific interference with viral infection mediated by molecules of homologous dsRNA operates in the cell interior and it not occasioned by any other inhibiting effect that might take place in vitro.

### Detailed description of the figures

**Fig. 1.** Schematic representation of the location in the PMMoV, TEV and AMV genome of viral sequences which, following their in vitro transcription starting from the respective clones and hybridisation of ssRNA transcription products, gave rise to different dsRNAs used in the examples of interference with viral infection mediated by dsRNA.
**Fig. 2.** Specific interference with PMMoV infection by means of dsRNA in a local host.
   Response of *N. tabacum* cv. "Xanthi nc" to PMMoV inoculated alone (left half of the leaves) or inoculated together with 54-kDa dsRNA (A), antisense 54-kDa ssRNA (As) (B), TEV-HC dsRNA (C) or in vitro transcription buffer (D) (right half of the leaves, respectively). The leaves were photographed at 5 dpi. Some local lesions (arrows heads) are pointed out in (B), (C) and (D).
**Fig. 3.** Interference mediated by dsRNA with PMMoV infection in a systemic host.
   (A) Interference with PMMoV infection is dsRNA specific. Northern blot analysis of total RNA extracted from inoculated leaves (lines 1-6) or upper systemic leaves (lines 7-13) of N. benthamiana. The plants were inoculated either with the buffer alone (Mock), with PMMoV alone (-), or with PMMoV plus 54-kDa dsRNA, sense 54-kDa RNA (S), antisense 54-kDa RNA (As) or 54-kDa cDNA, as indicated. The leaf tissues were collected at 7 dpi, except for samples in lines 12 and 13, which were taken at 21 dpi. The samples in lines 2, 8 and 12 were taken from the same plant which showed no symptoms of disease throughout its life-cycle. The sample in line 13 was taken from another individual which showed symptoms of disease at 21 dpi. The 54-kDa dsRNA used in the inoculum was charged in line 14 for comparative purposes.
   (B) Interference with PMMoV infection is produced by means of different homologous dsRNAs. RNA was extracted from the tissue of upper leaves of plants inoculated with PMMoV alone (-), or with PMMoV plus 54-kDa dsRNA, 30-kDa RNA or TEV HC-dsRNA at 7 dpi, as indicated.
   (C) Interference with PMMoV infection requires a minimum length of dsRNA. RNA was extracted starting from inoculated leaf tissue (lines 1 and 4) or starting from upper leaf tissue (lines 2, 3, 5 and 6) of plants infected with PMMoV alone or with PMMoV plus 1/3 54-kDa dsRNA at 7 (lines 1, 2, 4 and 5) or at 12 dpi (lines 3 and 6). The 1/3 54-kDa dsRNA used in the inoculation was charged in line 7 for comparative purposes.
      Equivalent quantities (1 µg) of each sample of RNA were fractionated by electrophoresis in 1% agar gel in (A), (B) and (C), and then a probe was used marked with digoxigenin corresponding to 54-kDa RNA. Indicated in the margins are the positions of the PMMoV RNA and the RNA species, partially denaturalised, derived from dsRNA used in the inoculum. Indicated in the lower part are the bands of 25S ribosome RNA stained with ethidium bromide, as charge control for the gels.
**Fig. 4.** Interference mediated by dsRNA with infection by different plant viruses.
   (A) Interference mediated by dsRNA against TEV. Northern blot analysis of total RNA extracted from inoculated leaves (lines 1-4) or systemic leaves (lines 5-8) of N. tabacum inoculated with TEV alone or with TEV plus TEV-HC dsRNA at 6 dpi.
      Equivalent quantities (5 µg) of each sample of RNA were fractionated by electrophoresis in 1% agar gel and the filter was hybridised with an RNA probe marked with digoxigenin specific towards TEV. Indicated in the margins are the positions of the TEV RNA and the RNA species, partially denaturalised, derived from dsRNA used in the inoculum.
   (B) Interference mediated by dsRNA against AMV. Northern blot analysis of total RNA extracted from inoculated leaf (lines 1, 3 and 4) or systemic leaf (lines 6 and 7) of N. benthamiana plants inoculated with AMV RNAs 1, 2 and 3 alone (-) or with this mixture plus AMV-3 dsRNA, M, RNA extracted starting from plant inoculated with buffer. AMV RNAs 3 and 4 (line 2) and AMV-3 dsRNA (line 5) were charged in the gel for comparative purposes.
      Equivalent quantities (1 µg) of each sample of RNA were fractionated by electrophoresis in 1.2% agar gel and the filter was hybridised with an RNA probe marked with digoxigenin specific towards AMV RNA 3. Indicated in the margins are the positions of the AMV RNAs 3 and 4 and the RNA species, partially denaturalised, derived from dsRNA used in the inoculum. Indicated in the lower part are the bands of 25S ribosome RNA stained with ethidium bromide, as charge control for the gels.
**Fig. 5.** Transitory expression of 54-kDa dsRNA mediated by *Agrobacterium* interferes with PMMoV infection.
   (A) Plants of *N. benthamiana* were initially infiltrated, as indicated in the figure, with cultures of *A. tumefaciens* carriers of the sense (S) or antisense (As) 54-kDa RNA expression vector, or with a mixture of both cultures (S+As 54-kDa RNAs). After 4 days, the agroinfiltrated leaves of these plants were inoculated with PMMoV or with buffer (M).
   (B) After another 15 days, the accumulation of PMMoV RNA in inoculated leaves (lines 1-7) and in upper leaves (lines 8-14) of two plants per treatment were tested by means of Northern blot analysis.
      Equivalent quantities (1 µg) of each sample of RNA were fractionated by electrophoresis in 1% agar gel and the filter was hybridised with an RNA probe marked with digoxigenin specific towards 54-kDa RNA. Indicated in the margin is the position of the PMMoV RNA. Indicated in the lower part are the bands of 25S ribosome RNA stained with ethidium bromide, as charge control for the gels.

## Claims

1. A method for degrading in a specific way any nucleotide sequence of single-strand RNA (ssRNA), preferably viral RNA, in the cells of any plant.

2. A method in accordance with claim 1, consisting of the direct application of double-strand RNA (dsRNA) molecules, with sufficient sequence similarity with any fragment of ssRNA that is wished to be degraded in the treated plant.

3. The direct application of exogenous dsRNA, in accordance with claim 2, in any tissue of the plant, preferably leaf, by any procedure, preferably inoculation or infiltration.

4. A dsRNA, in accordance with claims 2 and 3, of sufficient size, preferably of more than 500 base pairs, obtained by any natural or synthetic procedure, preferably mixing and hybridising complementary transcripts of ssRNA produced by means of in vitro transcription.

5. A method, in accordance with claims 1, 2, 3 and 4, for degrading any viral ssRNA in any plant, by means of the direct application of dsRNA possessing sequence similarity with any fragment of viral ssRNA.

6. A method, in accordance with claims 1, 2, 3 and 4, for degrading any messenger RNA (mRNA) of any endogenous gene or transgene of the plant, by means of the direct application of dsRNA possessing sequence similarity with any fragment of the target gene or transgene.

7. A method, in accordance with the above claims, for silencing the expression of any virus gene in the cells of a plant, consisting of the direct application of dsRNA in the cells of the plant, possessing sequence similarity with any fragment of the viral gene that is wished to be silenced in the treated plant.

8. A method, in accordance with the above claims, for silencing at the post-transcriptional level the expression of any endogenous gene or transgene in the cells of a plant, consisting of the direct application of dsRNA in the cells of the plant, possessing sequence similarity with any fragment of the endogenous gene or transgene that is wished to be silenced in the treated plant.

9. A method, in accordance with the above claims, by which the silencing of a transitory expression gene, preferably a virus gene, protects the plant from the disease caused by the virus.

10. A method, in accordance with the above claims, by which the silencing of an endogenous gene of the plant modifies a constitutive and observable phenotype character of the plant.

11. A method, in accordance with the above claims, by which the silencing of a transgene, introduced into the plant by genetic engineering, modifies an incorporated and observable phenotype character of the plant.
